# EUROPEAN PATENT APPLICATION

(11) **EP 4 765 140 A1**
(43) Date of publication of application: **24.06.2026**
(21) Application number: 25226631.7
(22) Date of filing: 22.12.2025
(51) Int. Cl.: G16H 10/60, G06N 3/0475, G16H 50/70

(54) **SYSTEM AND METHOD FOR SUMMARIZING HEALTH DATA FOR USE BY LARGE LANGUAGE MODELS**

(30) Priority: 23.12.2024 US 202419000209
(71) Applicant: GOOGLE LLC, Mountain View CA 94043 (US)
(72) Inventor: PATHAK, Anupam J., Mountain View, CA 94043 (US); GARRISON, Jake Henry, Mountain View, CA 94043 (US)
(74) Representative: Maikowski & Ninnemann Patentanwälte Partnerschaft mbB

(57) **Abstract**

Provided is a system for standardizing health data for use by machine-learned models in responding to user queries. A computing system obtains health data for a user from one or more health data sources. The computing system generates standardized health data, wherein the standardized health data is generated by converting the health data into a standardized format usable by a machine-learned model. The computing system receives a user health query from the user. The computing system generates a model input for the machine-learned model comprising the user health query and the standardized health data. The computing system provides the model input to the machine-learned model. The computing system receives a model output from the machine-learned model. The computing system transmits the model output for display to the user.

## Description

### PRIORITY CLAIM

The present application is based on and claims priority to United States Application Number 19/000,209 having a filing date of December 23, 2024. The present application claims priority to and the benefit of each of such applications.

### FIELD

The present disclosure relates generally to using health data to provide responses to user queries. More particularly, the present disclosure relates to standardizing health data for use as input to a generative machine-learned model when generating responses to user queries.

### BACKGROUND

Large language models have enabled users to quickly find useful information based on a variety of topics. One technique for broadening the ability of a large language model is to provide contextual information for the specific prompt that the model receives. However, given that large language models are largely trained on natural language content, any supplemental data that does not use natural language content may be difficult for a large language model to utilize.

### SUMMARY

Aspects and advantages of embodiments of the present disclosure will be set forth in part in the following description, or can be learned from the description, or can be learned through practice of the embodiments.

One example aspect of the present disclosure is directed to a computer-implemented method of summarizing health data for use by large language models. The computer-implemented method can comprise obtaining, by a computing system comprising one or more processors, health data for a user from one or more health data sources. The method can further comprise generating, by the computing system, standardized health data, wherein the standardized health data is generated by converting the health data into a standardized format usable by a machine-learned model. The method can further comprise receiving, by the computing system, a user health query from the user. The method can further comprise generating, by the computing system, a model input for the machine-learned model comprising the user health query and the standardized health data. The method can further comprise providing, by the computing system, the model input to the machine-learned model. The method can comprise receiving, by the computing system, model output from the machine-learned model. The method can further comprise transmitting, by the computing system, the model output for display to the user.

Another example aspect of the present disclosure is directed to one or more tangible non-transitory computer-readable media storing computer-readable instructions that when executed by one or more processors cause the one or more processors to perform operations. The operations can comprise obtaining health data for a user from one or more health data sources. The operations can further comprise generating standardized health data, wherein the standardized health data is generated by converting the health data into a standardized format usable by a machine-learned model. The operations can further comprise receiving a user health query from the user. The operations can further comprise generating a model input for the machine-learned model comprising the user health query and the standardized health data. The operations can further comprise providing the model input to the machine-learned model. The operations can further comprise receiving model output from the machine-learned model. The operations can further comprise transmitting the model output for display to the user.

Another example aspect of the present disclosure is directed to a computing system having one or more processors and one or more non-transitory computer-readable media storing instructions that when executed by the one or more processors cause the one or more processors to perform operations. The operations may comprise, for example, obtaining health data for a user from one or more health data sources. The operations can further comprise generating standardized health data, wherein the standardized health data is generated by converting the health data into a standardized format usable by a machine-learned model. The operations can further comprise receiving a user health query from the user. The operations can further comprise generating a model input for the machine-learned model comprising the user health query and the standardized health data. The operations can further comprise providing the model input to the machine-learned model. The operations can further comprise receiving model output from the machine-learned model. The operations can further comprise transmitting the model output for display to the user.

Other aspects of the present disclosure are directed to various systems, apparatuses, non-transitory computer-readable media, user interfaces, and electronic devices.

These and other features, aspects, and advantages of various embodiments of the present disclosure will become better understood with reference to the following description and appended claims. The accompanying drawings, which are incorporated in and constitute a part of this specification, illustrate example embodiments of the present disclosure and, together with the description, serve to explain the related principles.

### BRIEF DESCRIPTION OF THE DRAWINGS

Detailed discussion of embodiments directed to one of ordinary skill in the art is set forth in the specification, which makes reference to the appended figures, in which:
FIG. 1 depicts a diagram of a system that processes health data for use in responding to user queries with a large language model in accordance with example embodiments of the present disclosure;
FIG. 2 depicts an example data processing system in accordance with example embodiments of the present disclosure;
FIG. 3 depicts a query response system in accordance with example embodiments of the present disclosure;
FIG. 4 depicts a block diagram of an example computing system for standardizing health data for use by a large language model according to example embodiments of the present disclosure;
FIG. 5 depicts an example client-server environment according to example embodiments of the present disclosure;
FIG. 6 depicts a flow diagram representing a process for converting health data into a standardized format for use in generating responses to user health queries in accordance with example embodiments of the present disclosure;
FIG. 7 depicts a block diagram of an example processing flow for using machine-learned model(s) to process input(s) to generate output(s);
FIG. 8 is a block diagram of an example implementation of an example machine-learned model configured to process sequences of information;
FIG. 9 is a block diagram of an example technique for populating an example input sequence;
FIG. 10 is a block diagram of an example computing device that performs according to example embodiments of the present disclosure; and
FIG. 11 is a block diagram of an example computing device that performs according to example embodiments of the present disclosure.

Reference numerals that are repeated across plural figures are intended to identify the same features in various implementations.

### DETAILED DESCRIPTION

In general, the present disclosure is directed to a system that can access health data from a plurality of sources (e.g., different computing devices with sensors) and convert the health data into a form usable by a large language model. In particular, the disclosed technology is related to generating query responses about health data when that health data comes from a variety of health data sources. For example, many user computing devices include sensors that can generate health data, such as fitness bands, smart watches, wearable technology, smartphones, and a plurality of other computing devices. The health data produced by these user computing devices can have unconventional formats. Large language models have been trained using natural language data and thus can best utilize information when that information is provided in a natural language format. The generated health data can be the raw output of sensors or data processing based on that raw data but formatted so that the data does not include any natural language descriptions of the data. As a result, when this data is provided to machine-learned models as input, the machine-learned model has trouble interpreting the data and extracting useful information from this data.

In response to this issue, the present technology can access data from a large variety of devices and sensors. The system includes a data processing system that can receive a variety of data inputs as input and output standardized data formatted such that a large language model (or other machine-learned model) can effectively use the data as context for responding to a user query. For example, the output of the standardized data processing system can include written descriptions of average values for the health data during a particular time period as well as anomalous events that occur during that time period. For example, the output of the data processing system can determine an average range for the user's heartbeat during a particular week. The standardized health data can also include a description of any anomalous events that occurred during that week (e.g., an anomalous event can be an instance or time period in which the value of a particular piece of health data is outside the average range for that value) as well as the description of any circumstances that are associated with that anomalous event.

Once the health data has been converted into a standardized readable format for the large language model (or other machine-learned model), a query response system can receive a user health query from a user. The user health query can be a user's question about their health data. The query response system can access the standardized health data and provide the data, along with the user query, to the large language model (or another machine-learned model). The large language model (or other machine-learned model) can generate an output that includes a response to the user query based, at least in part, on the formatted health data provided. The model output can be provided to the user for display.

In an example, a user can generate a user health query for a query response system. For example, the user health query can ask, "Have I been getting more sleep in the last month since I got my new bed?" The query response system can, based on this user health query, access user health information that has been standardized and that is associated with the user's sleep period. Specifically, the query response system can access sleep data for the past month and sleep data for the past year for comparison. The query response system can generate a prompt that includes the user query and the relevant sleep data. The large language model (or other machine-learned model) can take the prompt as input. Based on the prompt, the large language model (or other machine-learned model) can analyze the standardized health data and the query to generate a response to the user. As an example, the response can include natural language text that reads: "Yes, your average amount of sleep has increased by 35 minutes in the last month compared to the prior year."

More particularly, a query response system can provide responses to input queries submitted via a computer network. In some examples, the user query can be directed towards health data that the user has specifically allowed the query response system to access. A variety of devices can produce health data. Specifically, health data can be produced by a variety of sensors included in a fitness band, a wearable computing system, a smartwatch, a smartphone, sensor systems integrated into articles of clothing, medical technology that is connected to the user during particular times of the day or in particular locations (e.g., hospitals), and any other computing systems that include sensors that enable the device to capture health data for a user.

In an embodiment, the data produced by sensors included in computing devices (or the sensors themselves) can be a time series of values. Each value represents a measured amount at a particular time. Sensors can measure a variety of data, including user movement, acceleration, the number of calories burned, the heart rate of the user, heart rate variability of the user, skin temperature, the presence or lack of moisture on the skin, and so on.

As discussed above, the time series for a particular value can include a measured value (e.g., the magnitude of a particular measurement) for each time step of a series of time steps. The time series data can be referred to as raw data. The raw data can be analyzed to generate a variety of other information, including sleep time, exercise levels, sleep quality, sleep patterns, step counts, and other information.

The data generated by these sensors can then be provided to a data processing system. The data processing system can be a system that takes raw data (or the results of analyzing raw data such as sleep quality) and converts the raw data into a standardized format that is easier for large language models (and other machine-learned models) to use. In some examples, a machine-learned model can be trained for a particular type of health data. For example, a machine-learned model can be trained to receive the raw data of a particular type as input and output data in the standardized format. A plurality of machine-learned models can be trained, one for each type of data that can be input. Thus, a query response system that provides responses to queries about health data can train a machine-learned model for each sensor type and health data type that the model expects to receive. In addition, a general machine-learned model can be trained to take, as input, health data from a sensor for which there is not an already trained model and output standardized health data.

In some examples, the standardized output can include data that describes the average range of values for a particular type of data for a variety of different time periods (hour, day, week, month, year, and so on) in a natural language format. Once the average range values have been determined, the data can also include anomalous events for that type of value that is outside the average range. In some examples, different time periods can have different average ranges. In general, a description of an anomalous event can include the longest time for which the event is an anomalous event. For example, suppose a particular anomalous event is outside the average range for the most recent hour, day, and week but not outside the average range for the most recent year or month. In that case, the description of that anomalous event can note that the anomalous event is outside the range for the most recent week (e.g., the largest time period for which the anomalous event is outside the average range).

Information about the average values of any anomalous event can be written in a natural language format. Large language models are trained to receive natural language descriptions of things and events as input. Thus, large language models can understand and make use of health data when the health data is formatted such that it is written in a natural language format.

Once the data processing system has converted the raw health data into a format readable via a machine-learned large language model (or other machine-learned model), that information can be used as input to a machine-learned model when the machine-learned model receives health-related queries from a user.

For example, a user can provide specific health data to the query response system. The query response system can use the health data to generate standardized health data in a format readable by a large language model (or another machine-learned model). The user can then submit a user query to the query response system. The query response system can access the standardized health data and provide the data to the large language model (or another machine-learned model) as context for the user query.

In some examples, the query response system can selectively access the health data based on the context of the user query. For example, if the user query is associated with a particular type of health data or with a particular time period, only the relevant health data may be accessed by the query response system for context in the large language model (or other machine-learned model). For example, if the user query is about health data from the last month, the query response system may only access standardized health data for the most recent month. In another example, if the user query is about variations in heart rate, the query response system may only access health data about the user's heart rate.

In this way, non-relevant health data may not be provided to the large language model (or other machine-learned model) as context for a query response. This reduces the overall size of the input to the large language model (or another machine-learned model). For example, the query response system can generate a prompt based on the user query and the relevant standardized health data. The prompt can be provided to the large language model (or other machine-learned model) as input. The large language model (or other machine-learned model) can generate a model output based on the input prompt. That output can be a natural language response to the query input. In another example, the model output can be multimodal, including figures, video, or any other content useful to respond to the user health query.

The model output (e.g., query response) can be transmitted to the user computing device from which the user query was received. The user computing device can display the model response to the user. The user can ask follow-up or clarifying questions, and the query response system can generate further responses using the large language model (or another machine-learned model).

The systems, methods, devices, and/or computer-readable media (e.g., tangible non-transitory computer-readable media) in the disclosed technology can provide a variety of technical effects and benefits, including an improvement in the effectiveness with which large language models can provide accurate responses using health data generated by the computing device. In particular, by converting the health data to a standardized format that is more easily usable by the large language model (or other machine-learned model), the disclosed technology enables the large language model (or other machine-learned model) to generate more accurate responses while not increasing the cost of processing the user health query.

Additionally, the disclosed technology can use the user health query to filter or search the standardized health data. Only standardized health data that is determined to be relevant to the user health query is used as context to the machine-learned model. This reduces the size of the input to the machine-learned model. Reducing the size of the input increases the speed of generating the model output and reduces the cost of processing the input without increasing the cost. In this way, the query response system can improve the accuracy of query responses and improve user experience without increasing the cost.

With reference now to the figures, example embodiments of the present disclosure will be discussed in further detail.

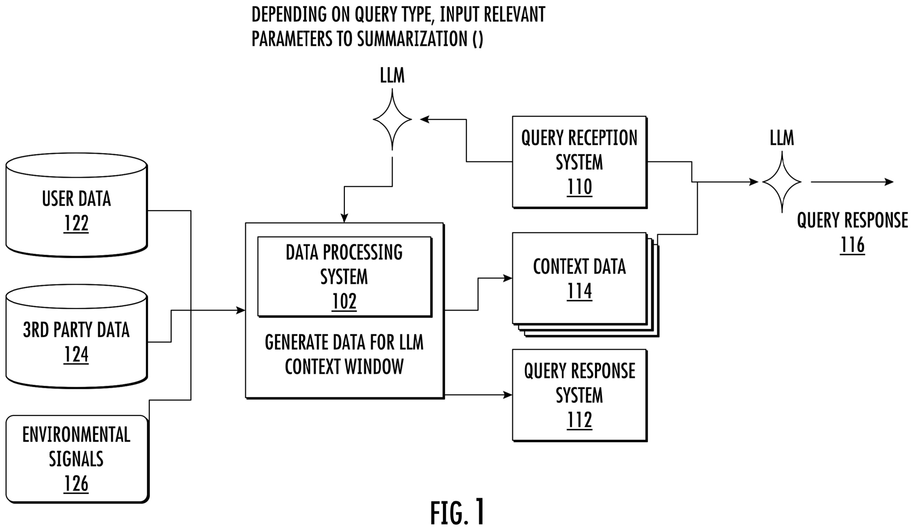

FIG. 1 depicts a diagram of a system that processes health data for use in responding to user queries with a large language model (or other machine-learned model) in accordance with example embodiments of the present disclosure. In this example, the system includes a data processing system 102, a query reception system 110, and a query response system 112. The data processing system 102 can receive data from one or more user data stores 122, one or more third-party data stores 124, and one or more environmental data signals 126. The query response system 112 and the query reception system 110 can include or use one or more large language models (e.g., a generative model or a sequence processing model).

The data processing system 102 can access user data from one or more user data stores 122. The data in the user data store 122 can include raw data received from one or more sensors in computing devices or connected to the user directly. The sensors can include accelerometers, gyroscopes, light sensors, pressure sensors, and temperature sensors. These sensors can be included in a variety of computing devices, including smartphones, fitness bands, smartwatches, health sensors that are directly connected to the user's skin, and so on.

The user data store 122 can also include data that is generated based on an analysis of the raw data. For example, the user data store 122 can include estimated sleep times that are based on user movement during sleep. The only data available in the user data store 122 is data that the user has agreed to make available to the data processing system 102. The third-party data store 124 can include information gathered by third parties. In some examples, the third-party data store 124 includes information provided by the user to a third-party application, such as a journaling application that the user permits the data processing system 102 to access.

Environmental data signals 126 can include information about the temperature, pressure, the number of daylight hours, the amount of light at a particular time and place, and so on. The environmental data signals 126 can be used to determine the environment near the user at a particular location and time. The environmental data signals 126 can include information about the user's location on various days and times and the environment the user would be experiencing at those locations.

The data processing system 102 can access data from the data sources and generate data with a standardized format based on the information received from these data stores. In some examples, the data processing system 102 can convert the data into a format that can be accessible to a large language model (or other machine-learned model). For example, the raw sensor data may include the information in a format that was difficult for the machine-learned models to understand and analyze correctly. As a result, if the machine-learned model attempts to generate an adequate response based on the raw health data, there is a higher chance of the model misunderstanding the raw health data. The data processing system 102 can convert the raw health data (e.g., sensor data) into a format that includes natural language explanations of the average range of values for particular measurements during particular periods of time as well as any anomalous events in which the values move outside the average ranges.

Using third-party data or environmental signals, the data processing system 102 can give context to anomalous events based on user-provided data and environmental data during the anomalous event(s). In this way, the proper contextual information can allow the large language model (or other machine-learned model) to answer user queries more effectively.

The query reception system 110 can receive a query from a user. Once the user health query has been received, the computing system can access data that has been converted into a standardized format and provided as context data 114. In some examples, a machine-learned model is used to identify the particular pieces of information relevant to the user query. In this way, the amount of context data 114 provided to the large language model (or other machine-learned model) as context for a user health query can be reduced. Reducing the total amount of context data 114 can allow the large language model (or other machine-learned model) to process the input more quickly and at less cost.

The query response system 112 can generate a prompt for the large language model (or other machine-learned model) that includes the user health query, and any context data 114 generated based on the standardized health data the data processing system 102 has generated. The prompt can be provided to the large language model 130. The large language model(s) 130 can generate a model output based on the input. The model output can include a query response 116. The query response 116 can include a natural language response to the user health query.

The model output can be provided and transmitted to the user computing device. The user computing device can display the model output to the user in response to the user health query.

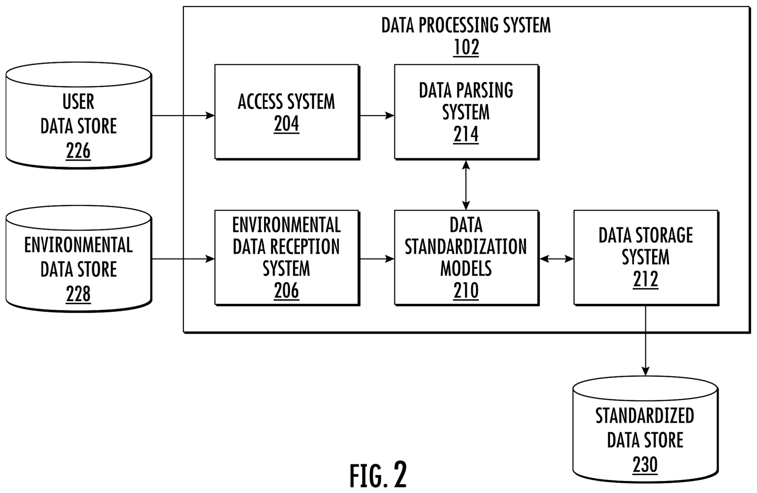

FIG. 2 depicts a data processing system in accordance with example embodiments of the present disclosure. The data processing system 102 includes an access system 204, a data parsing system 214, an environmental data reception system 206, one or more data standardization models 210, and a data storage system 212. The data processing system 102 can receive data from a user data store 226, an environmental data store 228, and output standardized data to a standardized data store 230.

The access system 204 can access health data from a user data store 226. The user data store 226 can include raw data captured from sensors associated with computing devices owned by the user, including smartwatches, smartphones, fitness bands, embedded systems capable of capturing health data, and any other computing systems capable of capturing health data using sensors.

In addition to raw data from sensors, the user data store 226 can include data from analyzing the raw sensor data. The sensors can provide movement data using an accelerometer or gyroscope, temperature data using a temperature sensor, tissue state data, tissue content data using a camera or other light measurement sensors, pressure sensors used to capture heart rate data, and so on. This data can be captured and used to determine information such as step count, distance traveled, calories burned, exercise time, heart rate variability, sleep duration, sleep quality, sleep stages, and other information that can be derived from the raw sensor data.

The access system 204 can access any health data the user has made available to the data processing system. In addition to data derived from the sensors included in a user computing device, the user health data can also include data from third-party applications such as mood ratings from the user, journal entries, and other information the user can access through their accounts on third-party systems. Once the access system 204 has accessed the health data from the user data store 226, the health data can be provided to the data parsing system 214.

In some examples, the data parsing system 214 can provide some preprocessing of the health data. In some examples, the data processing system 102 includes information on how to parse data from specific sensors or from specific applications. The data parsing system 214 can remove extraneous or unnecessary data in the health data and add useful information to enable the data standardization model(s) 210 to transform the data quickly and accurately into a standardized format.

In some examples, the environmental data reception system 206 can access data from the environmental data store 228. The environmental data store 228 can include temperature, air pressure, weather, and other information about the environment based on the user's location at particular dates and times. For example, if the user permits, the environmental data reception system 206 can access information with the user's location at various times and retrieve information about the environmental situation at each location and time. This environmental data can be matched with other health data and used as context when analyzing the health data.

The data processing system 102 can provide health and environmental data to one or more data standardization models 210. In some examples, there is a plurality of different data standardization models 210. Each data standardization model 210 can be trained to standardize data from a particular source type. For example, one data standardization model 210 can be trained to take sleep data as input and output standardized sleep data. Other data standardization models 210 can be trained to take heart rate variability data as input and output a standardized heart rate variability data record. In this way, a plurality of models can be trained, each with a specific data source in mind, and each model can then provide an accurate conversion from raw data into the standardized data format. In another example, one data standardization model 210 can be trained to take a variety of different types of data as input and provide standardized formatted data as output.

In some examples, the standardized formatted data can include, for each data type and for one or more time periods, an average range of the data type in that time period. In addition, the standardized formatted data can include information describing any anomalous events that are present in the data. The anomalous event(s) can include any instance in which a particular type of sensor data exceeded the average range of data.

In some examples, the anomaly data can describe the longest time period for which a particular anomalous event has exceeded an average range. For example, if a particular anomalous event exceeds a range for a value for the most recent year, most recent month, and most recent week, the text describing the anomalous event can explain the specific details of the anomalous event, including the measured values of the anomalous event, the time that it lasted, and the most significant time period for which it exceeded the average range. In these examples, the input can describe that the anomalous event exceeds the average for the year. In other examples, the description of the anomalous event can include information describing each time period for which the data exceeds the average range (not just the longest time period).

Once the machine-learned model(s) have converted the data into a standardized format, the standardized data can be provided to the data storage system 212. The data storage system 212 can store the data in a standardized data store for later access.

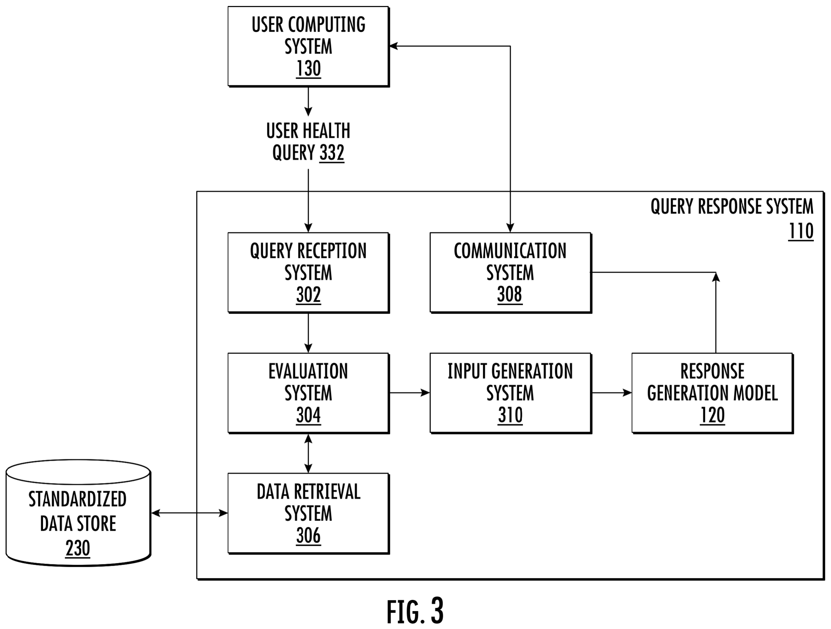

FIG. 3 depicts a query response system in accordance with example embodiments of the present disclosure. In this example, the query response system 112 can receive a user health query 332 from a user computing device. A user health query 332 can include a question from a user about the information included in their health data. The user health query 332 can specify a particular type of health data and a specific time range in which the user is interested. In some examples, the user health query 332 does not specify a particular type of data or specific time range, but that information can be inferred based on the user health query.

In some examples, the user health query 332 can include text or voice input from a user (e.g., a selection of a user interface element or a spoken question). In some examples, the user health query 332 can be received by the query reception system 302. The query reception system 302 receives the user health query 332 and provides the user health query 332 to an evaluation system 304.

The evaluation system 304 can analyze the user health query 332 to determine the particular type of health data that is needed to respond to the health query in the time frame required. For example, if the question is associated with the user's heart rate, the evaluation system 304 can determine that relevant information describes how often the user's heart beats and any associated information. Some information (e.g., the user's step count) can be determined to be irrelevant to the user query.

The evaluation system 304 can provide information about the topics and time frame needed by the user health query 332 to a data retrieval system 306. The data retrieval system 306 can access the relevant data from the standardized data store 230. In some examples, the data retrieval system 306 can determine whether the standardized data store 230 includes data associated with the topic and time frame associated with the health query. If so, that data can be retrieved. However, if the data retrieval system 306 determines that some of the data stored in the standardized data store 230 is not relevant, the data retrieval system 306 can refrain from retrieving the irrelevant data.

If no relevant data is identified in the standardized data store 230, the data retrieval system can transmit a report indicating a lack of relevant data to the evaluation system 304. Based on the available data in the standardized data store 230, the evaluation system 304 can determine one or more pieces of data that should be accessed. The evaluation system 304 can notify the user that the data necessary to respond to the query is unavailable in the standardized data store 230. The user can then update their query or provide more information that can be used in the standardized format. Once the data has been retrieved by the data retrieval system 306, the data can be provided by the evaluation system 304 to the input generation system 310.

The input generation system 310 can generate a model input based on the user health query 332, any relevant health information retrieved by the data retrieval system 306 from the standardized data store 230, and any contextual information necessary to adequately generate a model response. The model input can be provided to the response generation model 120. The response generation model 120 can generate a model output. The model output can then be transmitted to the user computing device for display to the user using the communication system 308.

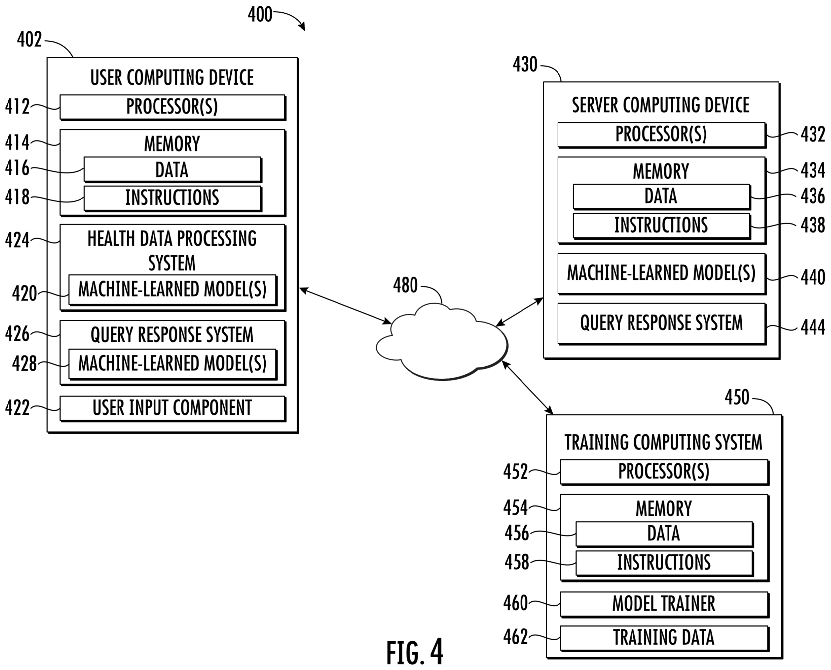

FIG. 4 depicts a block diagram of an example computing system 400 for standardizing health data for use by a large language model (or other machine-learned model) according to embodiments of the present disclosure. The computing system 400 includes a user computing device 402, a server computing system 430, and a training computing system 450 that are communicatively coupled over a network 480.

The user computing device 402 can be any type of computing device, such as a personal computing device (e.g., laptop or desktop), a mobile computing device (e.g., smartphone or tablet), a wearable computing device, an embedded computing device, or any other type of computing device.

The user computing device 402 includes one or more processors 412 and a memory 414. The one or more processors 412 can be any suitable processing device (e.g., a processor core, a microprocessor, an application specific integrated circuit (ASIC), a field-programmable gate array (FPGA), a controller, a microcontroller, etc.) and can be one processor or a plurality of processors that are operatively connected. The memory 414 can include one or more non-transitory computer-readable storage mediums, such as RAM, ROM, EEPROM, EPROM, flash memory devices, magnetic disks, etc., and combinations thereof. The memory 414 can store data 416 and instructions 418, which are executed by the processor 412 to cause the user computing device 402 to perform operations.

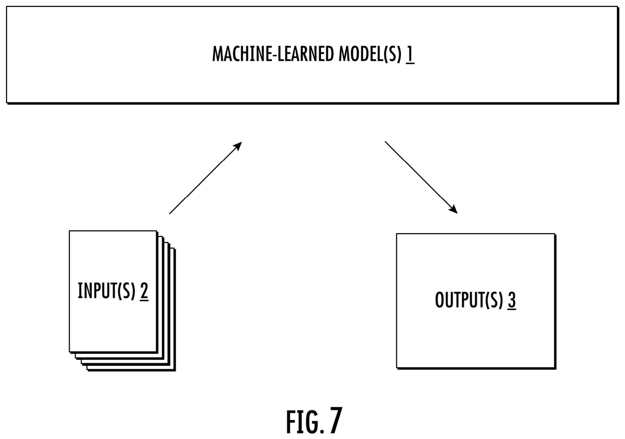

In some implementations, the user computing device 402 can store or include one or more machine-learned models (e.g., models 420 or 428) (e.g., a data processing model, response generation model, and so on). For example, the machine-learned models (e.g., models 420 or 428) can be or otherwise include various machine-learned models such as neural networks (e.g., deep neural networks) or other types of machine-learned models, including non-linear models and/or linear models. Neural networks can include feed-forward neural networks, recurrent neural networks (e.g., long short-term memory recurrent neural networks), convolutional neural networks or other forms of neural networks. Example machine-learned models (e.g., models 420 or 428) are discussed with reference to FIGS. 7-11.

In some implementations, the machine-learned model(s) (e.g., models 420 or 428) can be received from a server computing system 430 over network 480, stored in the memory 414 of the user computing device 402, and then used or otherwise implemented by the one or more processors 412. In some implementations, the user computing device 402 can implement multiple parallel instances of the machine-learned models (e.g., models 420 or 428).

More particularly, the health data processing system 424 can process health data into a standardized format and the query response system 426 can generate responses to user health queries using the standardized health data. To do so, the health data processing system 424 can access health data from a plurality of sensors and other data sources. As described above, the devices can include sensors that can capture raw data associated with this health data, including movement, temperature, pressure changes, the contents of blood or tissue using light analysis, and so on. The health data can also include information that is the result of the analysis of this raw sensor data. For example, the information can include information describing the amount of movement or exercise that a user experiences in a particular period based on movement data and heart rate data, sleep quality data, and any other data that can be generated based on analysis of the raw sensor data.

The health data processing system 424 can also access environmental data describing the temperature, pressure, humidity, and amount of daylight for the locations where the user is at a particular time. This data can be provided to the machine-learned model(s) (e.g., models 420 or 428). These machine-learned models can be trained to take data as input and output data that fits a standardized format. The standardized format can include a plurality of entries, the entries including an average range of values for a particular time period (e.g., an hour, a day, a week, a month, a year, etc.) and also describing any anomalous events that occurred during that time. Information about the environment can also be used to understand particular anomalous events or average ranges. For example, the temperature during a specific period of time may be very cold, and the user may have a very small number of steps. However, an anomalous event can be a warm day on which the user takes above an average number of steps. Thus, the environmental data for the location can be useful in analyzing the step counts of the user.

The machine-learned model(s) (e.g., models 420 or 428) can output a document that includes a list of averages for different values and anomalous events that occur during particular time periods. In some examples, the output can include natural language descriptions of these averages and any anomalous events. The natural language descriptions are easier for large language models to use as input.

Once the health data has been converted into a standardized format, it can be used by the query response system 426 to respond to user health queries. For example, a query response system 426 can receive a user health query from a user. The user health query is a question the user has about their health data. The user health query can include a natural language description of the question.

Once the query response system 426 has received the user health query, the query response system determines what particular health data is needed to respond to the query. Thus, the user health query can be filtered or searched to identify data associated with the correct topic and at the correct time. Selecting only the relevant health data allows the machine-learned model to operate more quickly and efficiently, reducing the cost and time needed to generate an accurate response. Once the query response system 426 has identified the relevant health data, the query response system can provide input to the machine-learned model 428, including the user health query and the selected standardized data. The machine-learned model 428 can output a model output that responds to the user health query based on the context available in the user health data.

The model output can include a natural language response to the user health query. In some examples, the model output can be multimodal, including text and images. The model output can reference specific information on the user's health data, and the user can then review the data themselves based on their own health data. The model output can be displayed to the user on the user computing device.

The user computing device 402 can also include one or more user input components 422 that receive user input. For example, the user input component 422 can be a touch-sensitive component (e.g., a touch-sensitive display screen or a touchpad) that is sensitive to the touch of a user input object (e.g., a finger or a stylus). The touch-sensitive component can serve to implement a virtual keyboard. Other example user input components include a microphone, a traditional keyboard, or other means by which a user can provide user input.

The server computing system 430 includes one or more processors 432 and a memory 434. The one or more processors 432 can be any suitable processing device (e.g., a processor core, a microprocessor, an ASIC, an FPGA, a controller, a microcontroller, etc.) and can be one processor or a plurality of processors that are operatively connected. The memory 434 can include one or more non-transitory computer-readable storage mediums, such as RAM, ROM, EEPROM, EPROM, flash memory devices, magnetic disks, etc., and combinations thereof. The memory 434 can store data 436 and instructions 438 which are executed by the processor 432 to cause the server computing system 430 to perform operations.

In some implementations, the server computing system 430 includes or is otherwise implemented by one or more server computing devices. In instances in which server computing system 430 includes plural server computing devices, such server computing devices can operate according to sequential computing architectures, parallel computing architectures, or some combination thereof.

As described above, the server computing system 430 can store or otherwise include one or more machine-learned models 440 (e.g., a large language model (or other machine-learned model), a sequence processing model, or another generative model). For example, the model(s) 440 can be or can otherwise include various machine-learned models. Example machine-learned models include neural networks or other multi-layer non-linear models. Example neural networks include feed forward neural networks, deep neural networks, recurrent neural networks, and convolutional neural networks. Example models 440 are discussed with reference to FIGS. 7-11.

The server computing system 430 can also include a query response system 444. The query response receives a user health query. It responds to the user health query based on standardized formatted data produced by the health data processing system (not shown herein). The query response system 444 can transmit the response to the user computing device for display.

The user computing device 402 and/or a server computing system 430 can train the models 420, 428, and/or 440 via interaction with the training computing system 450, which is communicatively coupled over the network 480. The training computing system 450 can be separated from or a portion of the server computing system.

The training computing system 450 includes one or more processors 452 and a memory 454. The one or more processors 452 can be any suitable processing device (e.g., a processor core, a microprocessor, an ASIC, an FPGA, a controller, a microcontroller, etc.) and can be one processor or a plurality of processors that are operatively connected. The memory 454 can include one or more non-transitory computer-readable storage mediums, such as RAM, ROM, EEPROM, EPROM, flash memory devices, magnetic disks, etc., and combinations thereof. The memory 454 can store data 456 and instructions 458 which are executed by the processor 452 to cause the training computing system 450 to perform operations. In some implementations, the training computing system 450 includes or is otherwise implemented by one or more server computing devices.

The training computing system 450 can include a model trainer 460 that trains the machine-learned models (e.g., models 420, 428, and/or 440) stored at the user computing device 402 and/or the server computing system 430 using various training or learning techniques, such as, for example, backwards propagation of errors. For example, a loss function can be backpropagated through the model(s) to update one or more parameters of the model(s) (e.g., based on a gradient of the loss function). Various loss functions can be used such as mean squared error, likelihood loss, cross entropy loss, hinge loss, and/or various other loss functions. Gradient descent techniques can be used to iteratively update the parameters over a number of training iterations.

In some implementations, performing backwards propagation of errors can include performing truncated backpropagation through time. The model trainer 460 can perform a number of generalization techniques (e.g., weight decays, dropouts, etc.) to improve the generalization capability of the models being trained.

In particular, the model trainer 460 can train the machine-learned models (e.g., models 420, 428, and/or 440) based on a set of training data 462. The training data 462 can include, for example, reference health input queries, query responses, raw health data, standardized health data, and so on. In some examples, the model trainer 460 can use query response evaluation data (or other feedback) from an evaluation system.

The model trainer 460 includes computer logic utilized to provide desired functionality. The model trainer 460 can be implemented in hardware, firmware, and/or software controlling a general-purpose processor. For example, in some implementations, the model trainer 460 includes program files stored on a storage device, loaded into a memory and executed by one or more processors. In other implementations, the model trainer 460 includes one or more sets of computer-executable instructions stored in a tangible computer-readable storage medium such as RAM hard disk or optical or magnetic media.

The network 480 can be any type of communications network, such as a local area network (e.g., intranet), wide area network (e.g., Internet), or some combination thereof and can include any number of wired or wireless links. In general, communication over the network 480 can be carried via any wired and/or wireless connection, using a wide variety of communication protocols (e.g., TCP/IP, Hypertext Transfer Protocol (HTTP), SMTP, FTP), encodings or formats (e.g., HTML, XML), and/or protection schemes (e.g., VPN, secure HTTP, SSL).

The machine-learned models described herein may be used in a variety of tasks, applications, and/or use cases. In some implementations, the input to the machine-learned model(s) of the present disclosure can include audio data. The machine-learned model(s) can process the audio data to generate an output based on a request. As an example, the machine-learned model(s) can process the audio data and generate a response that includes audio data by extracting information from the audio data and updating or modifying it based on the request.

In some implementations, the input to the machine-learned model(s) of the present disclosure can be text or natural language data. The machine-learned model(s) can process the text or natural language data to generate an output. As an example, the machine-learned model(s) can process the natural language data for a particular user health query and generate a prompt based on the user health query.

As discussed above, the input to the machine-learned model(s) of the present disclosure can be speech data. The machine-learned model(s) can process the speech data to generate an output. As an example, the machine-learned model(s) can process the speech data to generate a speech recognition output. The output of the speech recognition system can be used as input to the image generation model.

It should be understood that FIG. 4 illustrates an example computing system that can be used to implement the present disclosure. Other computing systems can be used as well. For example, in some implementations, the user computing device 402 can include the model trainer 460 and the training data 462. In such implementations, the model(s) 420 can be trained and used locally at the user computing device 402. In some implementations, the user computing device 402 can implement the model trainer 460 to personalize the model 420 based on user-specific data.

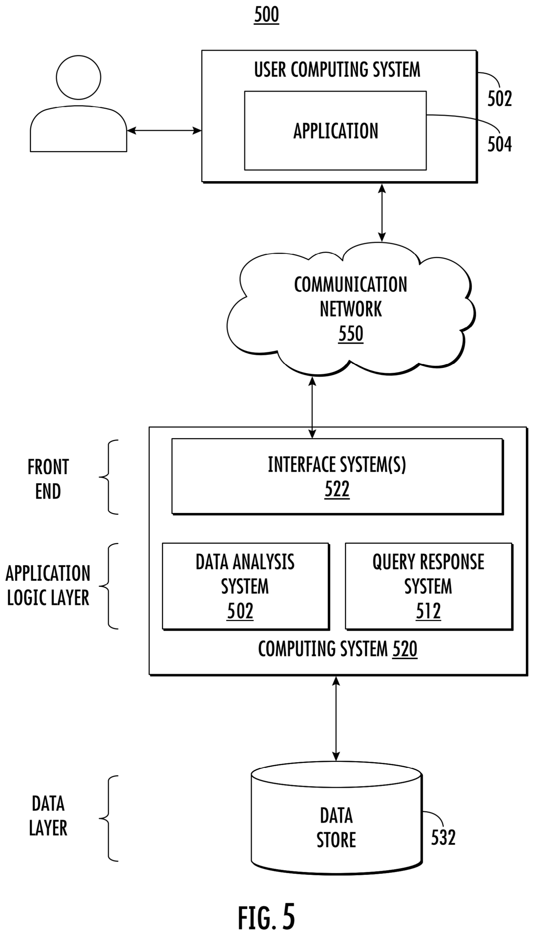

FIG. 5 depicts an embodiment of a client-server environment 500 according to the present disclosure. The client-server system environment 500 includes one or more user computing systems 502 and a server computing system 520. One or more communication networks 550 can interconnect these components. The communication network(s) 550 may be any of a variety of network types, including local area networks (LANs), wide area networks (WANs), wireless networks, wired networks, the Internet, personal area networks (PANs), or a combination of such networks.

The user computing system(s) 502 can be one of, but is not limited to, a personal computing system, a smartphone, a smartwatch, a laptop computing device, and a tablet computing system. In some examples, the user computing system 502 can include one or more application(s) 504, such as search applications, communication applications, navigation applications, productivity applications, game applications, word processing applications, or any other applications. The application(s) can include a health application. The health application can track user health data, aggregate that health data, provide analysis of the data, and respond to user queries about that data. For example, the user computing system 502 can, in response to user input, transmit a request to the server computing system 520. The request can be a user health query. The server computing system 520 can provide the user health query as input to a sequence processing model and return a model response to the user computing system 502.

As shown in FIG. 5, the server computing system 520 can generally be based on a three-tiered architecture, having a front-end layer, an application logic layer, and a data layer. As is understood by skilled artisans in the relevant computer and Internet-related arts, each component shown in FIG. 5 can represent a set of executable software instructions and the corresponding hardware (e.g., memory and processor) for executing the instructions. To avoid unnecessary detail, various components and engines that are not germane to conveying an understanding of the various examples have been omitted from FIG. 5. However, a skilled artisan will readily recognize that various additional components, systems, and applications may be used with the user computing system 502, such as that illustrated in FIG. 5, to facilitate additional functionality that is not specifically described herein. Furthermore, the various components depicted in FIG. 5 may reside on a single server computer or may be distributed across several server computers in various arrangements. Moreover, although server computing system 520 is depicted in FIG. 5 as having a three-tiered architecture, the various examples of embodiments are not limited to this architecture.

As shown in FIG. 5, the front end can include an interface system(s) 522, which receives communications from one or more user computing system 502 and communicates appropriate responses to the user computing system 502. For example, the interface system(s) 522 may receive requests in the form of HTTP requests, or other web-based application programming interface (API) requests. The user computing system 502 may be executing conventional web browser applications or applications developed for a specific platform to include any of a wide variety of computing devices and operating systems.

As shown in FIG. 5, the data layer can include a data store 532. The data store 532 can store the data used to produce responses to user health queries. In some examples, the data store 532 can represent a plurality of distinct databases, each database storing different data. For example, the data store 532 can include raw data from a plurality of sensors, environmental data, third-party data, and standardized formatted health data that has been processed to be accessible to a large language model (or another machine-learned model).

The application logic layer can include application data that provides a wide range of other applications and services, allowing users to submit queries and receive responses. The application logic layer can include a data analysis system 502 and a query response system 512.

Prior to a user submitting a user health query, the user can make their health data available to the server computing system 520. In some examples, the server computing system can be associated with a particular device, such as a smartphone or a smartwatch. The health data can include any data captured by the associated device and any health data that can be generated based on the captured sensor data of the user computing device. For example, a fitness band can include a sensor that determines the user's heart rate, movement, sleep quality, and so on. If the user chooses, they can make this data available to the server computing system 520. If the user makes health data available, the server computing system 520 can convert that data into a standardized format. Specifically, a data processing system 102 can access data provided by the user and output standardized formatted health data.

In some examples, the data processing system 102 can access the raw data, determining a specific machine-learned model that has been trained to generate a standardized version of the raw data, and provide the raw data to the appropriate machine-learned model. The machine-learned model can receive the raw data as input and generate model output. The model output can include data that has been standardized into a particular format to include an average range of values for a particular type of data and any anomalous events that occur within that time. The average data range in a particular time range can be described in natural language. For example, the standardized data can state, "The average range for your heart rate this week was between 65 beats per minute and 95 beats per minute."

Once the raw data has been standardized and stored in the data store 532, users can submit user health queries to the server computing system 520. The user health query can be provided by the interface system 522 to the query response system 512. The query response system 512 can analyze the user health query to determine which standardized data is useful in responding to the query. For example, the query response system 512 can search the data store 532 based on information included in the user health query. The query response system can select user health data associated with a particular type of data measurement or a specific time range. The selected data can be provided as input to the query response system 512.

The query response system 512 can generate input that includes the user health query and the retrieved standardized health data. The query response system 512 can provide the prompt to a large language model or other machine-learned model. The large language model (or other machine-learned model) can provide the model output, including a response to the user health query. The model output can also include references to the specific health data from which the response was generated. In this way, the user can verify that the response is based on accurate data.

Once the query response system 512 receives the model output from the large language model (or other machine-learned model), the query response system 512 can transmit the output to the user computing system 502. The user computing system 502 can display the model output to the user as necessary.

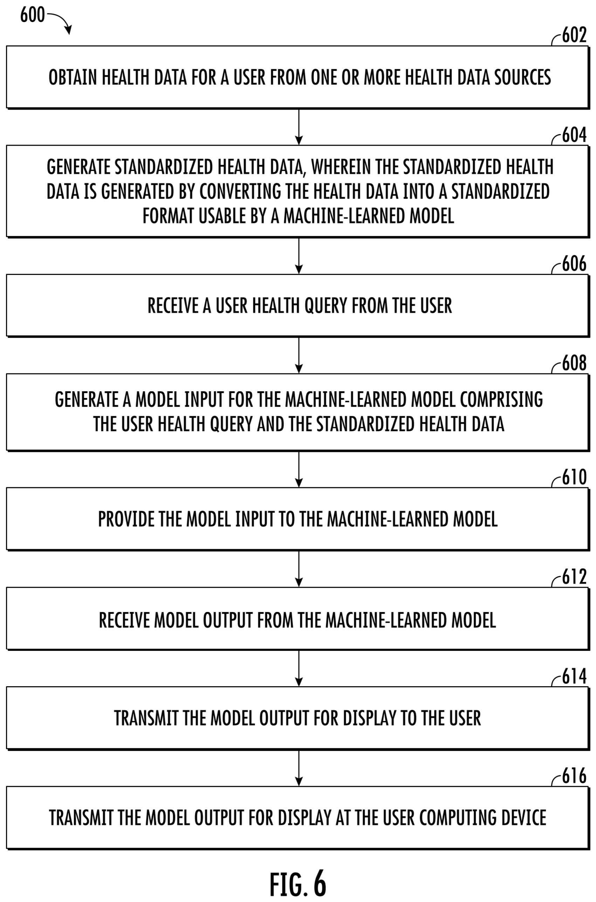

FIG. 6 is a flow diagram representing a process for converting health data into a standardized format for use in generating responses to user health queries in accordance with example embodiments of the present disclosure. The process can be performed by a computing system. The computing system can include one or more processors and one or more non-transitory computer-readable media that store instructions. The computing system can include a data processing system and a query response system. The data processing system can, at 602, obtain health data for a user from one or more health data sources. In some examples, the health data is produced by a plurality of distinct user computing devices.

In some examples, the plurality of distinct user computing devices can include, but is not limited to one or more of a smartphone, a fitness band, a smartwatch, an embedded computing system, a wearable computing device, a tablet, a laptop, etc. The data processing system can, at 604, automatically generate standardized health data, wherein the standardized health data is converted into a standardized format usable by a large language machine-learned model. The large language machine-learned model can be a generative large language machine-learned model.

In some examples, the data processing system can provide health data during a first time period from a first source from one or more health data sources to a first data processing machine-learned model trained to generate standardized health data based on data produced by the first source. The data processing machine-learned model can be referred to as a data standardization model. The data processing system can receive, from the first data processing machine-learned model, the first model output, wherein the first model output comprises standardized health data representing the health data during the first time period from the first source from the one or more health data sources. The data processing system can provide health data during a second time period from a second source from the one or more health data sources to a second data processing machine-learned model trained to generate standardized health data based on data produced by the second source.

The data processing system can receive, from the second data processing machine-learned model, the second model output, wherein the second model output includes standardized health data representing the health data during the first time period from the second source from the one or more health data sources. In some examples, automatically generating standardized health data comprises providing health data during a first time period from a first source from the one or more health data sources to a first data processing machine-learned model trained to generate standardized health data based on data produced by the first source.

The data processing system can receive, from the first data processing machine-learned model, the first model output, wherein the first model output comprises standardized health data representing the health data during the first time period from the first source from the one or more health data sources. In some examples, the first data processing machine-learned model is the same model as the second data processing machine-learned model. In other examples, the first data processing machine-learned model and the second data processing machine-learned model are different machine-learned models, each trained to take health data from different sources as input.

The data processing system can combine the first model output and the second model output into the standardized health data. In some examples, the standardized format for health data can be usable by a large language machine-learned model that includes natural language descriptions of anomalous events within the health data. In some examples, the natural language descriptions of anomalous events within the health data comprise the anomalous event that occurred and time data describing when the anomalous event occurred.

In some examples, the standardized format usable by a large language machine-learned model can include natural language descriptions of an average range for a particular health characteristic within the health data during a particular time period. The anomaly data for a particular health characteristic can be described based on the average value for that characteristic. In some implementations, a particular anomalous event for the particular health characteristic can be described with regard to an average value for the particular health characteristic. For example, the anomaly data can show that the user's heart rate exceeded its weekly average range for a certain time period on a certain day (e.g., for 2 hours on Monday, July 23).

In some examples, the query response system can, at 606, receive a user health query from the user. In some examples, the query response system can, at 608, generate a model input for the large language machine-learned model including the user health query and the standardized health data. The query response can, at 610, provide the model input to the large language machine-learned model.

In some examples, a user health query includes a natural language prompt about one or more health characteristics of the user. In some examples, the natural language prompt specifies a time period for the user health query. The query response system can filter the standardized health data based on the user health query. In some examples, the machine-learned model is a generative model. In some examples, the query response system can select a subset of standardized health data from the standardized health data based on the user health query. The query response system can generate the model input for the machine-learned model comprising the subset of standardized health data and the user health query.

In some examples, the query response system can, at 612, receive a model output from the large language machine-learned model. In some examples, the query response system can, at 614, transmit the model output for display to the user. The user computing device can, at 616, transmit the model output for display to the user.

FIG. 7 is a block diagram of an example processing flow for using machine-learned model(s) 1 to process input(s) 2 to generate output(s) 3 according to the present disclosure.

For example, as shown, machine-learned model(s) 1 can be or include one or multiple machine-learned models or model components. Example machine-learned models can include neural networks (e.g., deep neural networks). Example machine-learned models can include non-linear models or linear models. Example machine-learned models can use other architectures in lieu of or in addition to neural networks. Example machine-learned models can include decision tree-based models, support vector machines, hidden Markov models, Bayesian networks, linear regression models, k-means clustering models, etc.

The machine-learned model(s) 1 can be or include, or otherwise be representative of any of the one or more of the machine-learned models described above with respect to the preceding figures. For example, the machine-learned model(s) 1 can be or include, or otherwise be representative of a message generation model. Although various features, variations, and implementations described below are described with respect to machine-learned model(s) 1, it is to be understood that such features, variations, and implementations are to be understood as described with respect to the message generation model, etc., any other machine-learned component described herein.

Example neural networks can include feed-forward neural networks, recurrent neural networks (RNNs), including long short-term memory (LSTM) based recurrent neural networks, convolutional neural networks (CNNs), diffusion models, generative-adversarial networks, or other forms of neural networks. Example neural networks can be deep neural networks. Some example machine-learned models can leverage an attention mechanism such as self-attention. For example, some example machine-learned models can include multi-headed self-attention models.

The machine-learned model(s) 1 can include a single, or multiple instances of the same model configured to operate on data from input(s) 2. Machine-learned model(s) 1 can include multiple different models or multiple different model portions configured to operate on data from input(s) 2.

The machine-learned model(s) 1 can include an ensemble of different models that can cooperatively interact to process data from input(s) 2. For example, a model ensemble can include multiple models that have different attributes (e.g., different architectures, trained with different recipes, etc.). The ensemble can output an overall output based on the individual outputs of the constituent models. In this manner, for instance, the diverse constituent models can work together to provide system-level robustness by effectively aggregating over individual strengths and weaknesses of any given model. The respective individual outputs can be combined in a weighted combination, using a voting or routing mechanism, or a learned output layer (e.g., one or more feedforward or fully-connected layers).

The machine-learned model(s) 1 can employ a mixture-of-experts structure. See, e.g., Zhou et al., Mixture-of-Experts with Expert Choice Routing, arXiv:2202.09368v2 (Oct. 14, 2022). For example, different portions of a model can learn (explicitly or implicitly) different expertise areas, with pathways through the model being selected by a learned routing mechanism that engages the appropriate expert for a given input (e.g., a given portion of an input, such as on a per-token basis). For example, a feedforward network can be sparsely activated for a given portion of an input based on an output of a routing mechanism that processes the portion of the input. In this manner, for instance, the group of activated weights can form an "expert" that is selected by the router. On each forward pass, only a subset of the total model weights may be engaged, thereby decreasing a quantity of operations performed for processing a given input compared to a densely activated model. In this manner, for instance, the expressive and interpretive power of a high-parameter-count model can be achieved with more compute-efficient forward passes.

Input(s) 2 can generally include or otherwise represent various types of data. Input(s) 2 can include one type or many different types of data. Output(s) 3 can be data of the same type(s) or of different types of data as compared to input(s) 2. Output(s) 3 can include one type or many different types of data.

Example data types for input(s) 2 or output(s) 3 include natural language text data, software code data (e.g., source code, object code, machine code, or any other form of computer-readable instructions or programming languages), machine code data (e.g., binary code, assembly code, or other forms of machine-readable instructions that can be executed directly by a computer's central processing unit), assembly code data (e.g., low-level programming languages that use symbolic representations of machine code instructions to program a processing unit), genetic data or other chemical or biochemical data, image data, audio data, audiovisual data, haptic data, biometric data, medical data, financial data, statistical data, geographical data, astronomical data, historical data, sensor data generally (e.g., digital or analog values, such as voltage or other absolute or relative level measurement values from a real or artificial input, such as from an audio sensor, light sensor, displacement sensor, etc.), and the like. Data can be raw or processed and can be in any format or schema.

In multimodal inputs 2 or outputs 3, example combinations of data types include image data and audio data, image data and natural language data, natural language data and software code data, image data and biometric data, sensor data and medical data, etc. It is to be understood that any combination of data types in an input 2 or an output 3 can be present.

An example input 2 can include one or multiple data types, such as the example data types noted above. An example output 3 can include one or multiple data types, such as the example data types noted above. The data type(s) of input 2 can be the same as or different from the data type(s) of output 3. It is to be understood that the example data types noted above are provided for illustrative purposes only. Data types contemplated within the scope of the present disclosure are not limited to those examples noted above.

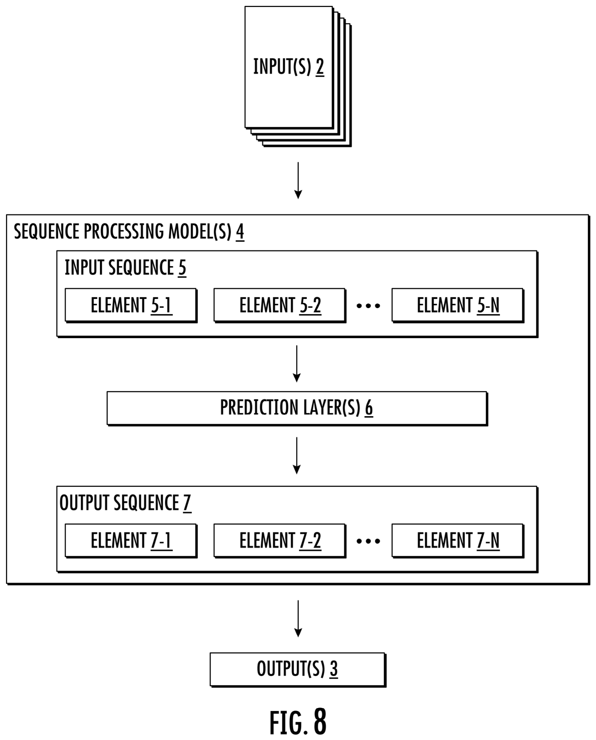

FIG. 8 is a block diagram of an example implementation of an example machine-learned model configured to process sequences of information according to the present disclosure. For instance, an example implementation of machine-learned model(s) 1 can include machine-learned sequence processing model(s) 4. An example system can pass input(s) 2 to sequence processing model(s) 4. Sequence processing model(s) 4 can include one or more machine-learned components. Sequence processing model(s) 4 can process the data from input(s) 2 to obtain an input sequence 5. Input sequence 5 can include one or more input elements 5-1, 5-2, . . . , 5-N, etc. obtained from input(s) 2. Sequence processing model 4 can process input sequence 5 using prediction layer(s) 6 to generate an output sequence 7. Output sequence 7 can include one or more output elements 7-1, 7-2, ... , 7-N, etc. generated based on input sequence 5. The system can generate output(s) 3 based on output sequence 7.

Sequence processing model(s) 4 can include one or multiple machine-learned model components configured to ingest, generate, or otherwise reason over sequences of information. For example, some example sequence processing models in the text domain are referred to as "Large Language Models," or LLMs. See, e.g., PaLM 2 Technical Report, Google, https://ai.google/static/documents/palm2techreport.pdf (n.d.). Other example sequence processing models can operate in other domains, such as image domains, see, e.g., Dosovitskiy et al., An Image is Worth 16x16 Words: Transformers for Image Recognition at Scale, arXiv:2010.11929v2 (Jun. 3, 2021), audio domains, see, e.g., Agostinelli et al., MusicLM: Generating Music From Text, arXiv:2301.11325v1 (Jan. 26, 2023), biochemical domains, see, e.g., Jumper et al., Highly accurate protein structure prediction with AlphaFold, 596 Nature 583 (Aug. 26, 2021), by way of example. Sequence processing model(s) 4 can process one or multiple types of data simultaneously. Sequence processing model(s) 4 can include relatively large models (e.g., more parameters, computationally expensive, etc.), relatively small models (e.g., fewer parameters, computationally lightweight, etc.), or both.

In general, the sequence processing model(s) 4 can obtain input sequence 5 using data from input(s) 2. For instance, the input sequence 5 can include a representation of data from input(s) 2 in a format understood by sequence processing model(s) 4. One or more machine-learned components of the sequence processing model(s) 4 can ingest the data from input(s) 2, parse the data into pieces compatible with the processing architectures of sequence processing model(s) 4 (e.g., via "tokenization"), and project the pieces into an input space associated with prediction layer(s) 6 (e.g., via "embedding").

Sequence processing model(s) 4 can ingest the data from input(s) 2 and parse the data into a sequence of elements to obtain input sequence 5. For example, a portion of input data from input(s) 2 can be broken down into pieces that collectively represent the content of the portion of the input data. The pieces can provide the elements of the sequence.

Elements 5-1, 5-2, ... , 5-N can represent, in some cases, building blocks for capturing or expressing meaningful information in a particular data domain. For instance, the elements can describe "atomic units" across one or more domains. For example, for textual input source(s), the elements can correspond to groups of one or more words or sub-word components, such as sets of one or more characters.

For example, elements 5-1, 5-2, ... , 5-N can represent tokens obtained using a tokenizer. For instance, a tokenizer can process a given portion of an input source and output a series of tokens (e.g., corresponding to input elements 5-1, 5-2, . . . , 5-N) that represent the portion of the input source. Various approaches to tokenization can be used. For instance, textual input source(s) can be tokenized using a byte-pair encoding (BPE) technique. See, e.g., Kudo et al., SentencePiece: A simple and language independent subword tokenizer and detokenizer for Neural Text Processing, Proceedings of the 2018 Conference on Empirical Methods in Natural Language Processing (System Demonstrations), pages 66-71 (October 31-November 4, 2018), https://aclanthology.org/D18-2012.pdf. Image-based input source(s) can be tokenized by extracting and serializing patches from an image.

In general, arbitrary data types can be serialized and processed into the input sequence 5. It is to be understood that element(s) 5-1, 5-2, ... , 5-N depicted in FIG. 8 can be the tokens or can be the embedded representations thereof.

Prediction layer(s) 6 can predict one or more output elements 7-1, 7-2, ... , 7-N based on the input elements. Prediction layer(s) 6 can include one or more machine-learned model architectures, such as one or more layers of learned parameters that manipulate and transform the input(s) to extract higher-order meaning from, and relationships between, input element(s) 5-1, 5-2, ... , 5-N. In this manner, for instance, example prediction layer(s) 6 can predict new output element(s) in view of the context provided by input sequence 5.

The prediction layer(s) 6 can evaluate associations between portions of the input sequence 5 and a particular output element. These associations can inform a prediction of the likelihood that a particular output follows the input context. For example, consider the textual snippet, "The carpenter's toolbox was small and heavy. It was full of ___." Example prediction layer(s) 6 can identify that "It" refers back to "toolbox" by determining a relationship between the respective embeddings. Example prediction layer(s) 6 can also link "It" to the attributes of the toolbox, such as "small" and "heavy." Based on these associations, prediction layer(s) 6 can, for instance, assign a higher probability to the word "nails" than to the word "sawdust."

A transformer is an example architecture that can be used in prediction layer(s) 6. See, e.g., Vaswani et al., Attention Is All You Need, arXiv:1706.03762v7 (Aug. 2, 2023). A transformer is an example of a machine-learned model architecture that uses an attention mechanism to compute associations between items within a context window. The context window can include a sequence that contains input sequence 5 and potentially one or more output element(s) 7-1, 7-2, ... , 7-N. A transformer block can include one or more attention layer(s) and one or more post-attention layer(s) (e.g., feedforward layer(s), such as a multi-layer perceptron).

Prediction layer(s) 6 can include other machine-learned model architectures in addition to or in lieu of transformer-based architectures. For example, recurrent neural networks (RNNs) and long short-term memory (LSTM) models can also be used, as well as convolutional neural networks (CNNs). In general, prediction layer(s) 6 can leverage various kinds of artificial neural networks that can understand or generate sequences of information.

Output sequence 7 can include or otherwise represent the same or different data types as input sequence 5. For instance, input sequence 5 can represent textual data, and output sequence 7 can represent textual data. Input sequence 5 can represent image, audio, or audiovisual data, and output sequence 7 can represent textual data (e.g., describing the image, audio, or audiovisual data). It is to be understood that prediction layer(s) 6, and any other interstitial model components of sequence processing model(s) 4, can be configured to receive a variety of data types in input sequence(s) 5 and output a variety of data types in output sequence(s) 7.

Output sequence 7 can have various relationships to input sequence 5. Output sequence 7 can be a continuation of input sequence 5. Output sequence 7 can be complementary to input sequence 5. Output sequence 7 can translate, transform, augment, or otherwise modify input sequence 5. Output sequence 7 can answer, evaluate, confirm, or otherwise respond to input sequence 5. Output sequence 7 can implement (or describe instructions for implementing) an instruction provided via input sequence 5.

Output sequence 7 can be generated autoregressively. For instance, for some applications, an output of one or more prediction layer(s) 6 can be passed through one or more output layers (e.g., softmax layer) to obtain a probability distribution over an output vocabulary (e.g., a textual or symbolic vocabulary) conditioned on a set of input elements in a context window. In this manner, for instance, output sequence 7 can be autoregressively generated by sampling a likely next output element, adding that element to the context window, and regenerating the probability distribution based on the updated context window, and sampling a likely next output element, and so forth.

Output sequence 7 can also be generated non-autoregressively. For instance, multiple output elements of output sequence 7 can be predicted together without explicit sequential conditioning on each other. See, e.g., Saharia et al., Non-Autoregressive Machine Translation with Latent Alignments, arXiv:2004.07437v3 (Nov. 16, 2020).

Output sequence 7 can include one or multiple portions or elements. In an example content generation configuration, output sequence 7 can include multiple elements corresponding to multiple portions of a generated output sequence (e.g., a textual sentence, values of a discretized waveform, computer code, etc.). In an example classification configuration, output sequence 7 can include a single element associated with a classification output. For instance, an output "vocabulary" can include a set of classes into which an input sequence is to be classified. For instance, a vision transformer block can pass latent state information to a multilayer perceptron that outputs a likely class value associated with an input image.

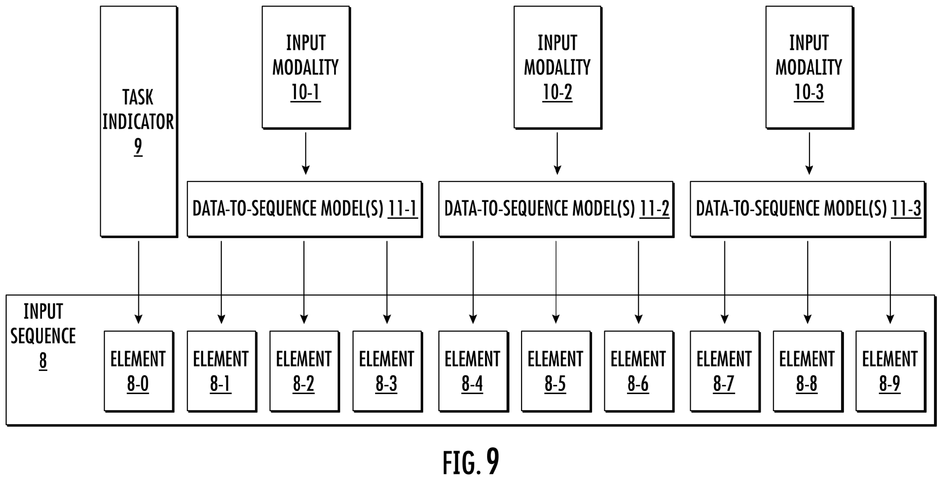

FIG. 9 is a block diagram of a technique for populating an example input sequence 8 according to the present disclosure. Input sequence 8 can include various functional elements that form part of the model infrastructure, such as an element 8-0 obtained from a task indicator 9 that signals to any model(s) that process input sequence 8 that a particular task is being performed (e.g., to help adapt a performance of the model(s) to that particular task). Input sequence 8 can include various data elements from different data modalities. For instance, an input modality 10-1 can include one modality of data. A data-to-sequence model 11-1 can process data from input modality 10-1 to project the data into a format compatible with input sequence 8 (e.g., one or more vectors dimensioned according to the dimensions of input sequence 8) to obtain elements 8-1, 8-2, 8-3. Another input modality 10-2 can include a different modality of data. A data-to-sequence model 11-2 can project data from input modality 10-2 into a format compatible with input sequence 8 to obtain elements 8-4, 8-5, 8-6. Another input modality 10-3 can include yet another different modality of data. A data-to-sequence model 11-3 can project data from input modality 10-3 into a format compatible with input sequence 8 to obtain elements 8-7, 8-8, 8-9.

Input sequence 8 can be the same as or different from input sequence 5. Input sequence 8 can be a multimodal input sequence that contains elements that represent data from different modalities using a common dimensional representation. For instance, an embedding space can have P dimensions. Input sequence 8 can be configured to contain a plurality of elements that have P dimensions. In this manner, for instance, example implementations can facilitate information extraction and reasoning across diverse data modalities by projecting data into elements in the same embedding space for comparison, combination, or other computations therebetween.

For example, elements 8-0, ... , 8-9 can indicate particular locations within a multidimensional embedding space. Some elements can map to a set of discrete locations in the embedding space. For instance, elements that correspond to discrete members of a predetermined vocabulary of tokens can map to discrete locations in the embedding space that are associated with those tokens. Other elements can be continuously distributed across the embedding space. For instance, some data types can be broken down into continuously defined portions (e.g., image patches) that can be described using continuously distributed locations within the embedding space.

In some implementations, the expressive power of the embedding space may not be limited to meanings associated with any particular set of tokens or other building blocks. For example, a continuous embedding space can encode a spectrum of high-order information. An individual piece of information (e.g., a token) can map to a particular point in that space: for instance, a token for the word "dog" can be projected to an embedded value that points to a particular location in the embedding space associated with canine-related information. Similarly, an image patch of an image of a dog on grass can also be projected into the embedding space. In some implementations, the projection of the image of the dog can be similar to the projection of the word "dog" while also having similarity to a projection of the word "grass," while potentially being different from both. In some implementations, the projection of the image patch may not exactly align with any single projection of a single word. In some implementations, the projection of the image patch can align with a combination of the projections of the words "dog" and "grass." In this manner, for instance, a high-order embedding space can encode information that can be independent of data modalities in which the information is expressed.

Task indicator 9 can include a model or model component configured to identify a task being performed and inject into input sequence 8, an input value represented by element 8-0 that signals which task is being performed. For instance, the input value can be provided as a data type associated with an input modality and projected along with that input modality (e.g., the input value can be a textual task label that is embedded along with other textual data in the input; the input value can be a pixel-based representation of a task that is embedded along with other image data in the input; etc.). The input value can be provided as a data type that differs from or is at least independent from other input(s). For instance, the input value represented by element 8-0 can be learned within a continuous embedding space.

Input modalities 10-1, 10-2, and 10-3 can be associated with various different data types (e.g., as described above with respect to input(s) 2 and output(s) 3).

Data-to-sequence models 11-1, 11-2, and 11-3 can be the same or different from each other. Data-to-sequence models 11-1, 11-2, and 11-3 can be adapted to each respective input modality 10-1, 10-2, and 10-3. For example, a textual data-to-sequence model can subdivide a portion of input text and project the subdivisions into element(s) in input sequence 8 (e.g., elements 8-1, 8-2, 8-3, etc.). An image data-to-sequence model can subdivide an input image and project the subdivisions into element(s) in input sequence 8 (e.g., elements 8-4, 8-5, 8-6, etc.). An arbitrary datatype data-to-sequence model can subdivide an input of that arbitrary datatype and project the subdivisions into element(s) in input sequence 8 (e.g., elements 8-7, 8-8, 8-9, etc.).

Data-to-sequence models 11-1, 11-2, and 11-3 can form part of machine-learned sequence processing model(s) 4. Data-to-sequence models 11-1, 11-2, and 11-3 can be jointly trained with or trained independently from machine-learned sequence processing model(s) 4. Data-to-sequence models 11-1, 11-2, and 11-3 can be trained end-to-end with machine-learned sequence processing model(s) 4.

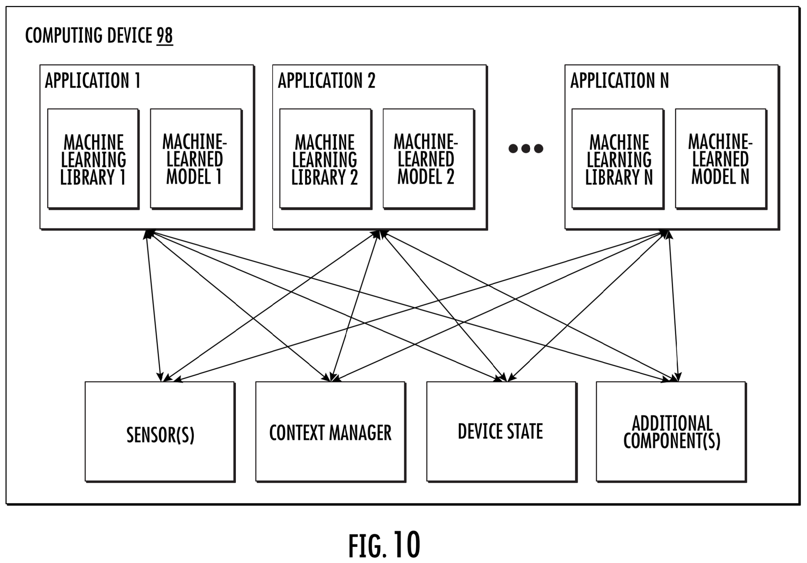

FIG. 10 is a block diagram of an example computing device 98 that performs according to example embodiments of the present disclosure. Computing device 98 can be a user computing device or a server computing device. For instance, computing device 98 can include a number of applications (e.g., applications 1 through N). Each application can contain its own machine-learned library and machine-learned model(s). For example, each application can include a machine-learned model. Example applications include a text messaging application, an email application, a dictation application, a virtual keyboard application, a browser application, etc. Each application can communicate with a number of other components of the computing device, such as, for example, one or more sensors, a context manager, a device state component, or additional components. In some implementations, each application can communicate with each device component using an API (e.g., a public API). In some implementations, the API used by each application is specific to that application.

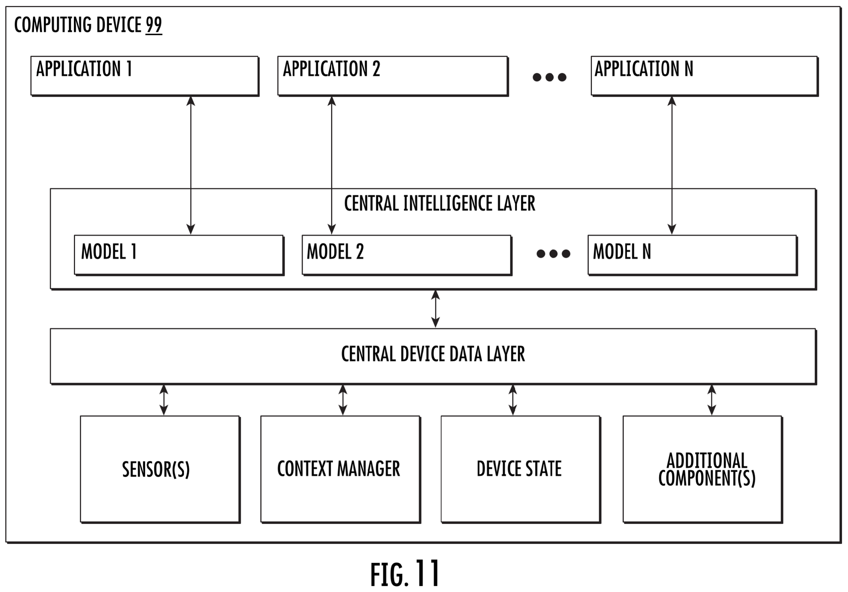

FIG. 11 is a block diagram of an example computing device 99 that performs according to example embodiments of the present disclosure. Computing device 99 can be the same as or different from computing device 98. Computing device 99 can be a user computing device or a server computing device. For instance, the computing device 99 can include a number of applications (e.g., applications 1 through N). Each application can be in communication with a central intelligence layer. Example applications include a text messaging application, an email application, a dictation application, a virtual keyboard application, a browser application, etc. In some implementations, each application can communicate with the central intelligence layer (and model(s) stored therein) using an API (e.g., a common API across all applications).

The central intelligence layer can include a number of machine-learned models. For example, in an embodiment, a respective machine-learned model can be provided for each application and managed by the central intelligence layer. In other implementations, two or more applications can share a single machine-learned model. For example, in some implementations, the central intelligence layer can provide a single model for all of the applications. In some implementations, the central intelligence layer is included within or otherwise implemented by an operating system of computing device 99.

The central intelligence layer can communicate with a central device data layer. The central device data layer can be a centralized repository of data for computing device 99. The central device data layer can communicate with a number of other components of the computing device, such as, for example, one or more sensors, a context manager, a device state component, or additional components. In some implementations, the central device data layer can communicate with each device component using an API (e.g., a private API).

Further to the descriptions above, a user may be provided with controls allowing the user to make an election as to both if and when systems, programs, or features described herein may enable collection of user information (e.g., information about a user's social network, social actions, or activities, profession, a user's preferences, or a user's current location), and if the user is sent content or communications from a server. In addition, certain data may be treated in one or more ways before it is stored or used, so that personally identifiable information is removed. For example, a user's identity may be treated so that no personally identifiable information can be determined for the user, or a user's geographic location may be generalized where location information is obtained (such as to a city, ZIP code, or state level), so that a particular location of a user cannot be determined. Thus, the user may have control over what information is collected about the user, how that information is used, and what information is provided to the user.

The technology discussed herein makes reference to servers, databases, software applications, and other computer-based systems, as well as actions taken, and information sent to and from such systems. The inherent flexibility of computer-based systems allows for a great variety of possible configurations, combinations, and divisions of tasks and functionality between and among components. For instance, processes discussed herein can be implemented using a single device or component or multiple devices or components working in combination. Databases and applications can be implemented on a single system or distributed across multiple systems. Distributed components can operate sequentially or in parallel.

While the present subject matter has been described in detail with respect to various specific example embodiments thereof, each example is provided by way of explanation, not limitation of the disclosure. Those skilled in the art, upon attaining an understanding of the foregoing, can readily produce alterations to, variations of, and equivalents to such embodiments. Accordingly, the subject disclosure does not preclude inclusion of such modifications, variations and/or additions to the present subject matter as would be readily apparent to one of ordinary skill in the art. For instance, features illustrated or described as part of one embodiment can be used with another embodiment to yield a still further embodiment. Thus, it is intended that the present disclosure cover such alterations, variations, and equivalents.

## Claims

**1.** A computer-implemented method, comprising:
obtaining, by a computing system comprising one or more processors, health data for a user from one or more health data sources;
generating, by the computing system, standardized health data, wherein the standardized health data is generated by converting the health data into a standardized format usable by a machine-learned model;
receiving, by the computing system, a user health query from the user;
generating, by the computing system, a model input for the machine-learned model comprising the user health query and the standardized health data;
providing, by the computing system, the model input to the machine-learned model;
receiving, by the computing system, model output from the machine-learned model; and
transmitting, by the computing system, the model output for display to the user.

**2.** The computer-implemented method of claim 1, wherein the health data is produced by one or more user computing devices, wherein, optionally, the one or more user computing devices comprise one or more of a smartphone, a smartwatch, a fitness band, a wearable computing device, a laptop, a tablet, or an embedded computing system.

**3.** The computer-implemented method of claim 1 or 2, wherein generating standardized health data comprises:
providing, by the computing system, health data during a first time period from a first health data source of the one or more health data sources to a first data processing machine-learned model trained to generate standardized health data based on data produced by the first health data source; and
receiving, from the first data processing machine-learned model, first model output, wherein the first model output comprises standardized health data representing the health data during the first time period from the first health data source from the one or more health data sources.

**4.** The computer-implemented method of claim 3, wherein generating standardized health data comprises:
providing, by the computing system, health data during a second time period from a second health data source from the one or more health data sources to a second data processing machine-learned model trained to generate standardized health data based on data produced by the second health data source; and
receiving, from the second data processing machine-learned model, second model output, wherein the second model output comprises standardized health data representing the health data during the first time period from the second health data source from the one or more health data sources.

**5.** The computer-implemented method of claim 4, wherein the first data processing machine-learned model is the same model as the second data processing machine-learned model and/or the method further comprises combining the first model output and the second model output into the standardized health data.

**6.** The computer-implemented method of any one of the preceding claims, wherein the standardized format usable by the machine-learned model comprises natural language descriptions of one or more anomalous events within the standardized health data.

**7.** The computer-implemented method of claim 6, wherein the natural language descriptions of the one or more anomalous events within the standardized health data comprises anomaly data representing an anomalous event within the one or more anomalous events that occurred and time data describing when the anomalous event occurred and/or the standardized format usable by the machine-learned model comprises natural language descriptions of an average range for a particular health characteristic within the health data.

**8.** The computer-implemented method of claim 7, wherein the anomalous event for the particular health characteristic is described with regard to an average value for the particular health characteristic.

**9.** The computer-implemented method of any one of the preceding claims, wherein the user health query comprises a natural language prompt including a question about one or more health characteristics of the user and/or the machine-learned model is a generative large language machine-learned model.

**10.** The computer-implemented method of claim 9, wherein the natural language prompt specifies a time period for the user health query and/or generating, by the computing system, the model input for the machine-learned model comprising the user health query and the standardized health data comprises filtering, by the computing system, the standardized health data based on the user health query.

**12.** A computing system, comprising:
one or more processors; and
one or more non-transitory computer-readable media that store instructions wherein, when executed by the one or more processors, the instructions cause the one or more processors to perform operations, the operations comprising:
obtaining health data for a user from one or more health data sources;
generating standardized health data, wherein the standardized health data is converted into a standardized format usable by a machine-learned model;
receiving a user health query from the user;
generating a model input for the machine-learned model comprising the user health query and the standardized health data;
providing the model input to the machine-learned model;
receiving model output from the machine-learned model; and
transmitting the model output for display to the user.

**13.** One or more non-transitory computer-readable media that collectively store instructions that, when executed by one or more computing devices, cause the one or more computing devices to perform operations, the operations comprising:
obtaining health data for a user from one or more health data sources;
generating standardized health data, wherein the standardized health data is converted into a standardized format usable by a machine-learned model;
receiving a user health query from the user;
generating a model input for the machine-learned model comprising the user health query and the standardized health data;
providing the model input to the machine-learned model;
receiving model output from the machine-learned model; and
transmitting the model output for display to the user.

**14.** The non-transitory computer-readable media of claim 13, wherein the health data is produced by a plurality of distinct user computing devices.

**15.** The non-transitory computer-readable media of claim 13 or 14, wherein generating the model input for the machine-learned model comprising the user health query and the standardized health data comprises:
selecting a subset of standardized health data from the standardized health data based on the user health query.
